# EUROPÄISCHE PATENTSCHRIFT

(11) **EP 1 194 005 B1**
(45) Veröffentlichungstag und Bekanntmachung des Hinweises auf die Patenterteilung: **20.01.2010**
(21) Anmeldenummer: 01810936.3
(22) Anmeldetag: 26.09.2001
(51) Int. Cl.: H04R 25/00, A61B 5/12, G06F 3/00, G06F 13/12, G06F 19/00

(54) **Verfahren zur Einstellung einer Übertragungscharakteristik einer elektronischen Schaltung**
Method for adjusting the transmission characteristics of an electronic circuit
Méthode de réglage de la caractéristique de transmission d'un circuit éléctronique

(30) Priorität: 27.09.2000 CH 18922000
(43) Veröffentlichungstag der Anmeldung: 03.04.2002
(73) Patentinhaber: Bernafon AG, 3018 Bern (CH)
(72) Erfinder: Lüdi, Christoph, 3048 Worblaufen (CH)
(74) Vertreter: Nielsen, Hans Jörgen Vind

(56) Entgegenhaltungen:
- EP-A- 0 915 639
- DE-A1- 4 221 300
- DE-A1- 4 418 203
- US-A- 5 835 611
- US-A- 5 923 764
- TAKAGI H ET AL: "IEC-based hearing aid fitting" SYSTEMS, MAN, AND CYBERNETICS, 1999. IEEE SMC '99 CONFERENCE PROCEEDINGS. 1999 IEEE INTERNATIONAL CONFERENCE ON TOKYO, JAPAN 12-15 OCT. 1999, PISCATAWAY, NJ, USA,IEEE, US, Bd. 3, 12. Oktober 1999 (1999-10-12), Seiten 657-662, XP010363689 ISBN: 0-7803-5731-0

## Beschreibung

Die Erfindung bezieht sich auf ein Verfahren zur Einstellung einer Übertragungscharakteristik einer elektronischen Schaltung gemäss Oberbegriff des unabhängigen Patentanspruchs. Das Verfahren lässt sich bspw. für die Einstellung von Hörgeräten anwenden.

Bei elektronischen Schaltungen zur Verarbeitung eines Eingangssignals in ein Ausgangssignal kann die Übertragungscharakteristik, bspw. die Verstärkung, von Parametern des Eingangssignals abhängen. Solche Eingangsparameter sind z. B. im Fall eines modulierten Eingangssignals die Amplitude oder die Frequenz des Eingangssignals. Eine beispielhafte Anwendung solcher Schaltungen liegt auf dem Gebiet der Hörgeräte. Ein modernes Hörgerät kann je nach dem individuellen Bedürfnis bzw. der individuellen Hörbehinderung seines Trägers eingestellt werden, wobei die Verstärkung einerseits von der Frequenz des Eingangssignals, andererseits vom Eingangsschallpegel abhängen soll.

Ein Verfahren zur Anpassung der Übertragungscharakteristik eines Hörgerätes an die Hörbehinderung seines Trägers ist im US-Patent Nr. 5,835,611 offenbart. Bei diesem Verfahren wird das einstellbare Hörgerät an einen Rechner (Personal Computer) mit Bildschirm und Maus angeschlossen. Auf dem Bildschirm wird eine Übertragungscharakteristik des Hörgerätes als Kurve in einem zweidimensionalen Diagramm dargestellt. Der Akustiker wählt mit der Computermaus ein Segment der zweidimensionalen Kurve aus und verändert die Übertragungscharakteristik durch Mausbewegungen, wodurch auch bestimmte die Übertragungscharakteristik definierende Betriebsparameter verändert werden. Auf diese Weise passt er die Übertragungscharakteristik einer gewünschten Zielcharakteristik an. Wenn eine genügende Anpassung erreicht ist, werden die angepassten Betriebsparameter an das Hörgerät übermittelt und dort gespeichert.

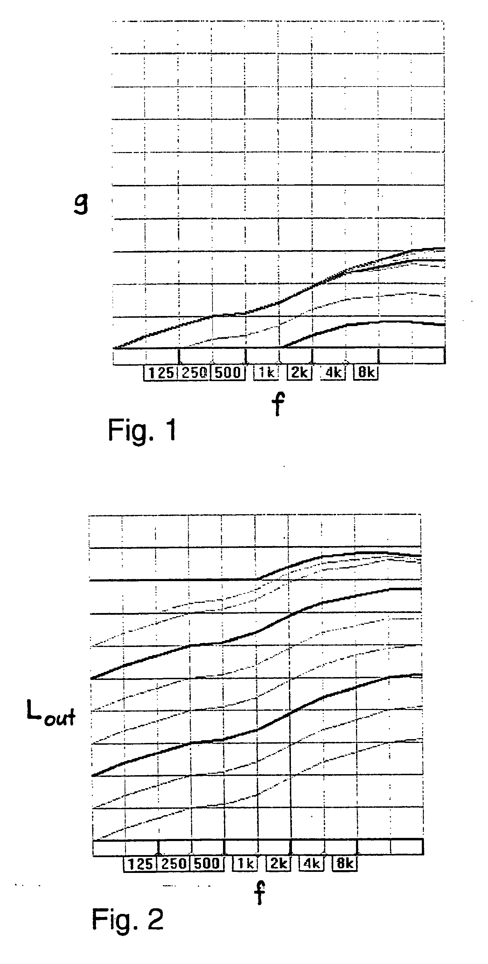

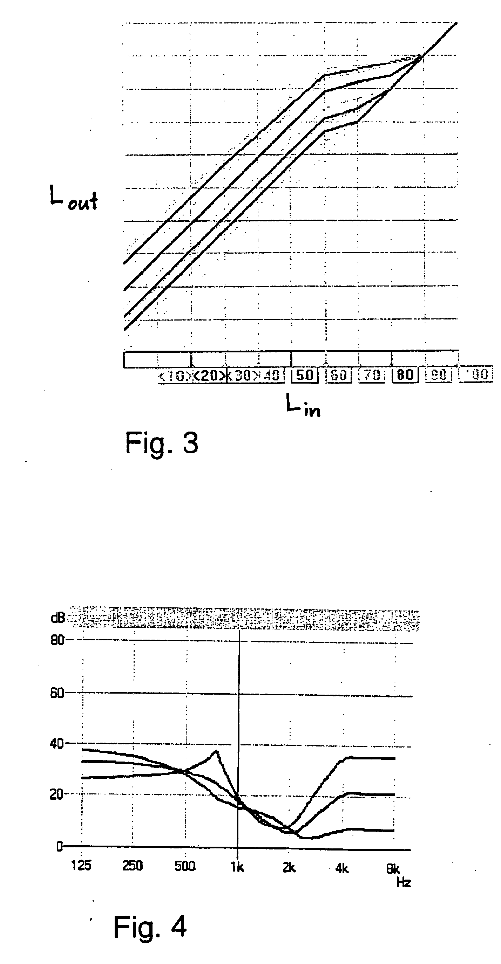

Für die Darstellung der Übertragungscharakteristik sind, je nach Hörgerätehersteller, zwei unterschiedliche zweidimensionale Diagramme gebräuchlich. Diese werden anhand der Figuren 1-3 erläutert.
(1) Im sogenannten Gain-view-Diagramm ist die Verstärkung g (**Figur 1**) bzw. der Ausgangsschallpegel Lₒᵤₜ (**Figur 2**) in Abhängigkeit von der Frequenz f aufgetragen. Weil die Verstärkung g bzw. der Ausgangsschallpegel Lₒᵤₜ zusätzlich noch vom Eingangsschallpegel abhängig ist, werden im selben Diagramm oftmals die Kurven für mehrere ausgewählte Eingangsschallpegel eingezeichnet.
(2) Im sogenannten Dynamic-view-Diagramm (**Figur 3**) ist der Ausgangsschallpegel Lₒᵤₜ in Abhängigkeit vom Eingangsschallpegel Lᵢₙ aufgetragen. Weil der Ausgangsschallpegel Lₒᵤₜ zusätzlich noch von der Frequenz abhängig ist, werden im selben Diagramm oftmals die Kurven für mehrere ausgewählte Frequenzen eingezeichnet.
Die Trennung zwischen den zwei Darstellungsweisen ist in der Denkweise der Benutzer, d. h. der Akustiker, begründet.

Das in der US-5,835,611 offenbarte Verfahren hat verschiedene Nachteile. Erstens wird entweder die Frequenz f oder der Schallpegel Lᵢₙ als Eingangssignal betrachtet. Während der Einstellung müssen oftmals beide Parameter g, Lᵢₙ gleichzeitig in Erwägung gezogen werden, was nicht oder allenfalls durch ein Hin- und Herschalten zwischen den beiden Darstellungsweisen möglich ist. Der logische Zusammenhang zwischen den Diagrammen muss im Kopf des Benutzers hergestellt werden, was ein grosses Mass an Vorstellungskraft bedingt. Es ist anzunehmen, dass dieser Zusammenhang nicht allen Benutzern klar ist. Zweitens sind die beiden Darstellungsweisen unübersichtlich. Wie nämlich oben beschrieben und in den Figuren 1-3 illustriert, werden im selben Diagramm mehrere Kurven eingetragen, welche sich jeweils durch den Wert eines weiteren Parameters Lᵢₙ, f unterscheiden (vgl. **Figur 4**). Jedem Parameterwert wird dabei eine Farbe zugewiesen, um eine Unterscheidung zu ermöglichen. Für den Benutzer ist es aber nicht einfach zu erfassen, welche Kurve zu welchem Parameter gehört; das Diagramm erscheint kompliziert. Je unregelmässiger die Hörgeräteeinstellung ist, desto verwirrender wird diese Art der Darstellung. Es ist absehbar, dass Einstellungen von Hörgeräten nächster Generationen nicht mehr vollständig mit den bekannten Diagrammen erfasst werden können.

Es ist Aufgabe der Erfindung, ein Verfahren zur Einstellung der Übertragungscharakteristik einer elektronischen Schaltung anzugeben, welches die obigen Nachteile nicht aufweist. Es soll insbesondere einfacher, übersichtlicher und benutzerfreundlicher sein als bekannte Verfahren. Die Aufgabe wird gelöst durch das Verfahren, wie es im ersten Patentanspruch definiert ist.

Die Erfindung basiert auf der Idee, zwei Parameter des Eingangssignals, im Fall eines Hörgerätes z. B. die Frequenz und den Eingangsschallpegel, als untrennbare Einheit zu behandeln. Aus diesen zwei Eingangsparametern werden Wertepaare gebildet, welche in einem bspw. kartesischen Koordinatensystem dargestellt werden. Die Einstellung der Übertragungscharakteristik weist jedem dieser Werte eindeutig einen Funktionswert, z. B. einen Ausgangspegel, zu. Wird der Funktionswert für jeden Punkt als Höhe basierend auf der Ebene dargestellt, ergibt sich eine (im Allgemeinen gekrümmte) Fläche im dreidimensionalen Raum. Diese Art der Visualisierung vereinigt die üblichen zweidimensionalen Diagramme in einer einzigen dreidimensionalen Darstellung, welche mehr Information über die Übertragungscharakteristik enthält und dabei übersichtlicher ist als die bisher bekannten zweidimensionalen Darstellungen. Durch Reduzieren des dreidimensionalen Diagramms auf Gitternetzlinien und Drehen in eine entsprechende Position kann das dreidimensionale Diagramm auf die bekannten zweidimensionalen Diagramme reduziert werden. Jede andere Ansicht zeigt eine dreidimensionale Darstellung, welche dem Benutzer eine neue Übersicht über die Übertragungscharakteristik eröffnet und die bekannten zweidimensionalen Diagramme verbindet. Der Übergang von einer zweidimensionalen Ansicht (bspw. Gain-view-Diagramm) in die andere (bspw. Dynamic-view-Diagramm) kann animiert erfolgen.

Im erfindungsgemässen Verfahren ist selbstverständlich auch eine indirekte Einstellung der Übertragungscharakteristik - bspw. über die Zielcharakteristik - mit inbegriffen.

Die Erfindung bietet einige Vorteile gegenüber den bekannten Verfahren:
- Lerneffekt: Durch den Übergang von einer zweidimensionalen Ansicht (bspw. Gain-view-Diagramm) in die andere (bspw. Dynamic-view-Diagramm) werden die beiden zweidimensionalen Diagramme verknüpft, wodurch ihre Darstellungsweisen und ihr Zusammenhang intuitiv erklärt wird.
- Rückwärtskompatibilität: Es ist möglich, die Übertragungscharakteristik nur in den bekannten zweidimensionalen Diagrammen darzustellen.
- Neue Anpassstrategien: Es ist möglich, genau ein Eingangssignal mit bestimmten Parameterwerten (z. B. eine bestimmte Frequenz und ein bestimmter Eingangsschallpegel) und die Übertragungscharakteristik für dieses Eingangssignal zu definieren. Ebenso können Bereiche ausgewählt und deren Übertragungscharakteristik definiert werden.
- Neue Manipulationstechniken: Durch die direkte Veränderung einer Fläche anstelle einzelner Kurven ergibt sich ein völlig neues Erscheinungsbild der Anpassung.

Diese Vorteile werden letztlich auch zu einer wirkungsvolleren Einstellung der Übertragungscharakteristik und, im Fall von Hörgeräten, zu einem besseren Hören sowie einer höheren Lebensqualität des Hörbehinderten führen.

Der Stand der Technik und die Erfindung werden anhand von Zeichnungen detailliert erläutert. Die hier diskutierten Beispiele beziehen sich ohne Einschränkung der Allgemeinheit auf die Einstellung von Hörgeräten; es sind aber auch andere Anwendungen des erfindungsgemässen Verfahrens möglich. In den Zeichnungen zeigen:
- Figur 1: ein Gain-view-Diagramm, wobei auf der Ordinate die Verstärkung eingetragen ist, gemäss Stand der Technik;
- Figur 2: ein Gain-view-Diagramm, wobei auf der Ordinate der Ausgangsschallpegel eingetragen ist, gemäss Stand der Technik;
- Figur 3: ein Dynamic-view-Diagramm gemäss Stand der Technik;
- Figur 4: ein Gain-view-Diagramm gemäss Stand der Technik;
- Figur 5: einen Aufbau zur Ausführung des erfindungsgemässen Verfahrens;
- Figur 6: ein Flussdiagramm mit dem Ablauf des erfindungsgemässen Verfahrens;
- Figur 7: eine erfindungsgemässe dreidimensionale Darstellung einer Übertragungscharakteristik; und
- Figuren 8: und 9 eine erfindungsgemässe dreidimensionale Darstellung zum Verändern der Übertragungscharakteristik.

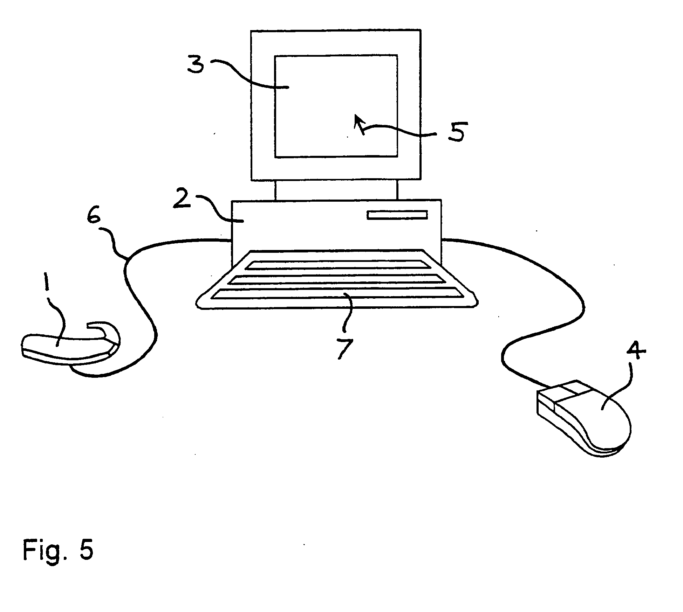

**Figur 5** zeigt einen möglichen Aufbau zur Ausführung des erfindungsgemässen Verfahrens. Der Aufbau beinhaltet einen Rechner 2, bspw. einen Personal Computer, mit einem Bildschirm 3 und einer Maus 4. Selbstverständlich kann statt der Maus 4 auch eine andere Zeigeeinrichtung wie z. B. ein Bitpad, ein Joystick, ein Trackball etc. verwendet werden. Mit der Maus 4 können ein Cursor 5 auf dem Bildschirm 3 positioniert und bestimmte Elemente des Bildschirminhaltes angewählt werden. An den Rechner 2 ist ein Hörgerät 1 angeschlossen, dessen Übertragungscharakteristik eingestellt werden soll, d. h. es besteht eine Datenübertragungsverbindung 6 zwischen dem Hörgerät 1 und dem Rechner 2. Die Datenübertragungsverbindung 6 kann als Kabel oder auf andere Weise, bspw. mittels Infrarotschnittstellen, ausgeführt sein. Eine Tastatur 7 für den Rechner 2 ist beim erfindungsgemässen Verfahren fakultativ, aber vorteilhaft.

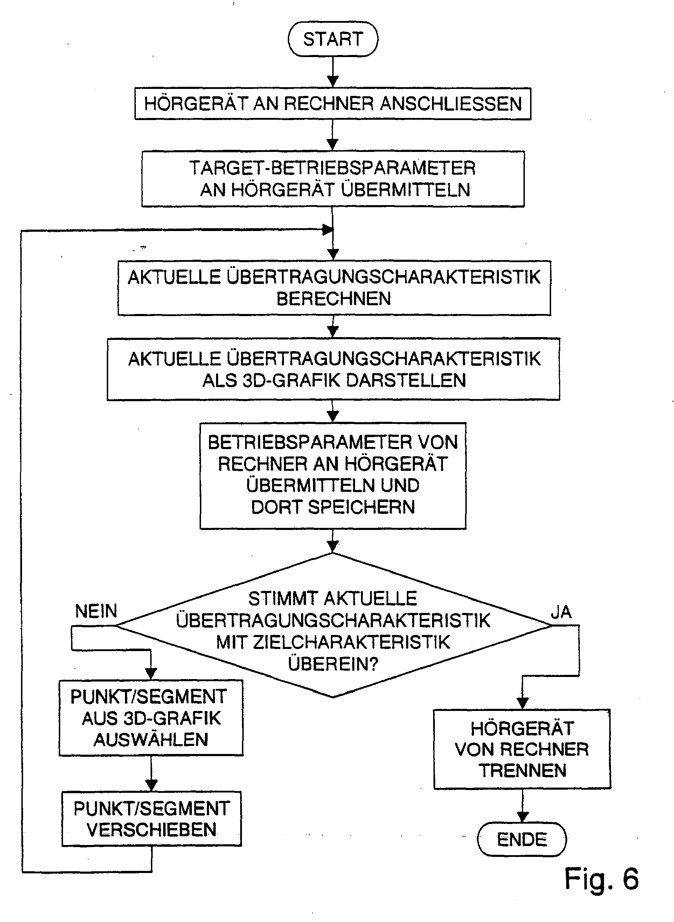

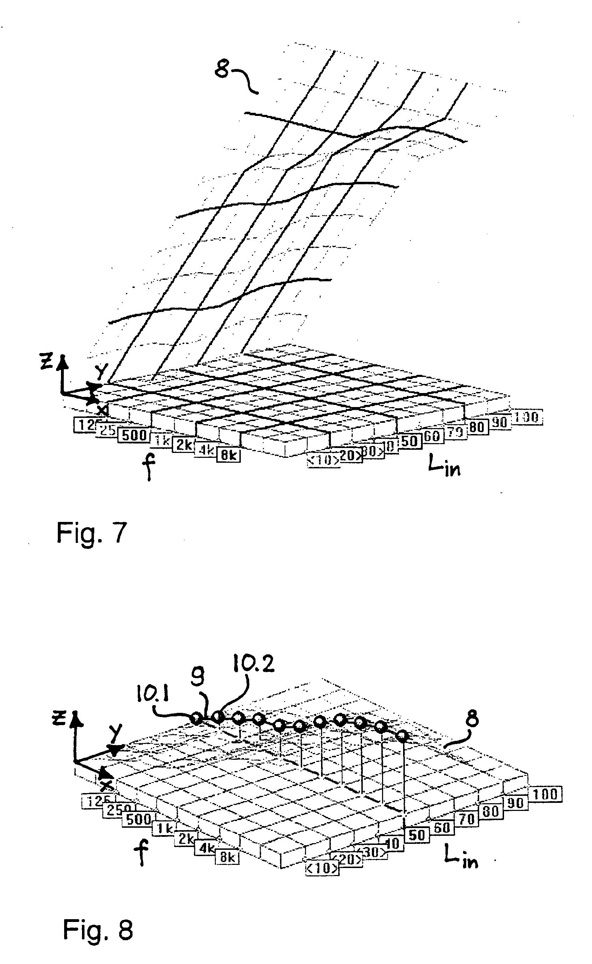

Im Flussdiagramm von **Figur 6** ist das erfindungsgemässe Verfahren am Beispiel einer Hörgeräteeinstellung schematisch dargestellt. Es wird ein Rechner 2 mit einem Bildschirm 3 und einer Maus 4 bereitgestellt, und das einzustellende Hörgerät 1 wird an den Rechner 2 angeschlossen. Somit ist der Aufbau von Fig. 5 eingerichtet. Das Hörgerät 1 beinhaltet (nicht dargestellte) Mittel zum Speichern von bestimmten Betriebsparametern, welche seine Übertragungscharakteristik definieren; solche Speichermittel können z. B. ein EEPROM-Speicher sein. In einem ersten Schritt wird der Rechner bspw. mit den Daten einer aktuellen Hörkurve des Hörbehinderten versehen. Aus diesen Daten wird eine Targetcharakteristik ermittelt, welche eine theoretisch optimale Verstärkungscharakteristik darstellt. Die Targetcharakteristik entspricht im Wesentlichen derjenigen Verstärkungscharkteristik welche rein rechnerisch die Hörkurve des Hörbehinderten so verstärkt, dass eine "normale" Hörkurve resultiert. Sie ist nicht zu verwechseln mit der Zielcharakteristik, welche auch angepasst ist an die subjektiven Höreindrücke und an die tatsächlichen Bedürfnisse des Hörbehinderten. Der Rechner 2 übermittelt die Targetcharakteristik als aktuelle Übertragungscharakteristik an das Hörgerät 1 und stellt diese gleichzeitig als dreidimensionale (3D-) Grafik auf dem Bildschirm 3 dar (vgl. Fig. 7). Nun beurteilt der Akustiker durch Tests mit dem Hörbehinderten, ob die aktuelle Übertragungscharakteristik mit einer von ihm angestrebten Zielcharakteristik übereinstimmt. Dies wird zunächst einmal kaum der Fall sein, weil ja jeder Hörbehinderte eine individuelle Hörschädigung und eine eigene subjektive Wahrnehmung hat. Der Akustiker wählt in diesem Fall mit Hilfe der Maus 4 und des Cursors 5 einen Punkt oder ein Segment der Grafik auf dem Bildschirm 3 aus und verändert die grafisch dargestellte Übertragungscharakteristik durch Verschieben der Maus 4 (vgl. Fig. 8). Dadurch werden bspw. auch die aktuellen Betriebsparameter verändert (oder es wird zumindest eine Vorschrift zum ändern bzw. Einstellen der aktuellen Betriebsparameter ermittelt). Die aktuellen Betriebsparameter können (nicht zwingend) vom Rechner 2 unmittelbar an das Hörgerät 1 übermittelt und in den dort vorhandenen Speichermitteln gespeichert werden. Weiter wird aus diesen Betriebsparametern eine neue aktuelle Übertragungscharakteristik berechnet und als 3D-Grafik auf dem Bildschirm 3 dargestellt. Dieser Anpassungsprozess wird so lange wiederholt, bis - innerhalb gewisser Toleranzgrenzen - der Hörbehinderte eine auch subjektiv realistische akustische Wahrnehmung aufweist. Das Hörgerät 1 kann danach vom Rechner 2 getrennt und vom Hörgeschädigten verwendet werden.

Ein Beispiel einer 3D-Grafik, wie sie im erfindungsgemässen Verfahren auf dem Bildschirm 3 dargestellt wird, ist in **Figur 7** gezeigt. Auf der x-Achse ist die Frequenz f (in Hz, logarithmische Skala), auf der y-Achse der Eingangsschallpegel Lᵢₙ (in dB) aufgetragen. Die z-Achse stellt den Ausgangsschallpegel Lₒᵤₜ oder die Verstärkung g dar, welche(r) eine Funktion der Frequenz und des Eingangsschallpegels ist. Vorzugsweise kann dabei die Darstellung zwischen dem Ausgangsschallpegel Lₒᵤₜ und der Verstärkung g soweit eventuell weiteren im Wesentlichen äquivalenten Charakteristiken, beispielsweise dem Offset (d.h. der Differenz) zur Targetcharakteristik, umgeschaltet werden. Die grafische Darstellung dieser Funktion ergibt eine (im Allgemeinen nicht ebene) Fläche 8 im dreidimensionalen Raum. Die Form dieser Fläche 8 ist charakteristisch für die jeweilige Hörgeräteeinstellung bzw. die individuelle Hörbehinderung des Hörgeräteträgers. Diese dreidimensionale Darstellung ist äusserst übersichtlich, intuitiv und einfach zu handhaben. Die Fläche braucht keine Fläche im wörtlichen Sinn zu sein sondern kann irgendein Gebilde im 3D-Raum darstellen. Selbstverständlich ist es möglich, mit bekannten Algorithmen den Blickwinkel zu verändern, d. h. die Grafik zu drehen. Dabei sind insbesondere zwei interessante Spezialfälle zu beachten:
- Blickt man parallel zur y-Richtung auf die Grafik, so sieht man das Gain-view-Diagramm wie in Fig. 1 bzw. 2 (bzw. äquivalent dazu den Offset zur Targetcharaktestik).
- Blickt man parallel zur x-Richtung auf die Grafik, so sieht man das Dynamic-view-Diagramm wie in Fig. 3 oder Fig. 4 bzw. ein äquivalentes Diagramm.

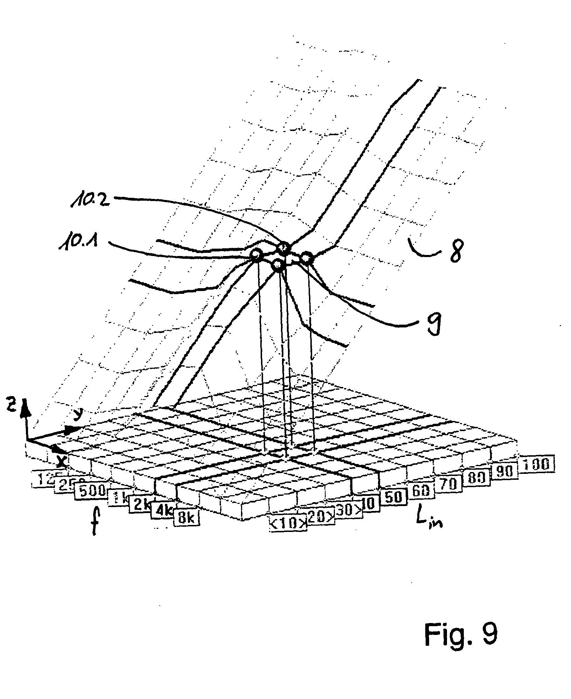

Die **Figuren 8** **und** **9** zeigen eine mögliche Art, die Übertragungscharakteristik zu verändern. Auf dem Bildschirm 3 wird die aktuelle Übertragungscharakteristik in Form einer Fläche 8 in einer 3D-Grafik dargestellt - ähnlich wie in Fig. 7 gezeigt, hier jedoch aus einer anderen Blickrichtung. Der Akustiker hat die Möglichkeit, mit der Maus 4 oder über die Tastatur 7 Teile der 3D-Grafik grafisch hervorzuheben.

Diese Teile entsprechen dann bspw. einem durch Einstellungsschritte zu manipulierenden Bereich 9 der Betriebsparameter. In der Figur 8 ist dieser Bereich 9 eine Kurve mit einem konstanten Eingangsparameter g bzw. Lᵢₙ- hier z. B. Eingangsschallpegel Lᵢₙ = 50 dB. In der Figur 9 entspricht er vier Punkten, welche ein Flächensegment begrenzen. Bestimmte Punkte des Bereichs 9 können mit grafischen Markierungen 10.1, 10.2, ... wie bspw. Kugeln markiert sein. Der Akustiker kann nun mit der Maus 4 eine dieser Markierungen 10.1, 10.2, ... anwählen und diese in z-Richtung beliebig verschieben. Dies bewirkt eine Veränderung der Übertragungscharakteristik und damit eine Formänderung der 3D-Fläche 8. Zur Ermittlung dieser veränderten Übertragungscharakteristik kann je nach dem noch eine Berechnung erfolgen. Eine solche kann bspw. eine numerische Filtersimulation der an sich bekannten Art für die Ermittlung der Frequenzabhängigkeit bei festem Eingangsschallpegel sein. In einem solchen Fall wird sich die Veränderung in einer Umgebung der angewählten Markierung am stärksten auswirken, während die Auswirkungen mit zunehmendem Abstand von der ausgewählten Markierung immer kleiner werden. Es soll hier noch erwähnt werden, dass die Markierungen 10.1, 10.2, ... verstellbare Betriebsparameter repräsentieren und nicht notwendigerweise Punkte der 3D-Fläche 8 sein müssen. Die Darstellung der Betriebsparameter durch diese Markierungen in der Grafik erfolgt aber vorzugsweise so, dass ein Verstellen eines Betriebsparameters die Übertragungscharakteristik dergestalt beeinflusst, dass die 3D-Fläche 8 der Markierung 10.1, 10.2, ... im Wesentlichen eng folgt oder dass diese sogar in der Fläche liegt. Es sei noch betont, dass vorstehend nur ein Beispiel dafür beschrieben wurde, wie die Übertragungs- und/oder Zielcharakteristik mit der Maus 4 verändert werden kann. Es sind auch andere Ausführungsarten möglich, z. B. das anfängliche Auswählen nur eines Punktes oder aller verfügbarer Betriebsparameter anstelle eines Bereiches 9 die Benutzung der Tastatur an Stelle der Maus.

## Patentansprüche

1. Verfahren zur Einstellung einer Übertragungscharakteristik einer elektronischen Schaltung (1), wobei
eine Berechnungs-Einrichtung (2) mit einer Anzeigeeinrichtung (3) und einer Einrichtung zur Auswahl, Markierung und/oder Veränderung (4) bereitgestellt wird,
eine Datenübertragungsverbindung (6) zwischen der einzustellenden elektronischen Schaltung (1) und der Berechnung Einrichtung (2) hergestellt wird,
eine aktuelle Übertragungscharakteristik der elektronischen Schaltung (1) durch eine Grafik auf der Anzeigeeinrichtung (3) repräsentiert wird,
mit Hilfe der Einrichtung zur Auswahl, Markierung und/oder Veränderung (4) bei Bedarf ein Punkt (10.1, 10.2, ...) oder ein Segment der Grafik auf der Anzeigeeinrichtung (3) ausgewählt wird und die grafisch dargestellte Übertragungscharakteristik verändert wird, wodurch auch eine Vorschrift zur Veränderung bestimmter, die Übertragungcharakteristik definierender Betriebsparameter ermittelt wird, und
die veränderten Betriebsparameter an die elektronische Schaltung (1) übermittelt und dort gespeichert werden,
**dadurch gekennzeichnet, dass**
die Übertragungscharakteristik der elektronischen Schaltung (1) als Gebilde (8) in einer dreidimensionalen Grafik auf der Anzeigeeinrichtung (3) dargestellt wird.

2. Verfahren nach Anspruch 1, wobei die elektronische Schaltung (1) ein Eingangssignal zu einem Ausgangssignal verarbeitet und in der dreidimensionalen Grafik ein Parameter (g, Lₒᵤₜ) des Ausgangssignals als Funktion von zwei Parametern (f, Lᵢₙ) des Eingangssignals dargestellt wird.

3. Verfahren nach Anspruch 1 oder 2, wobei eine Zielcharakteristik vorgegeben wird und die aktuelle Übertragungscharakteristik dieser Zielcharakteristik schrittweise angepasst wird.

4. Verfahren nach einem der Ansprüche 1-3, wobei zwecks Wahl einer geeigneten Ansicht die dreidimensionale Grafik gedreht wird.

5. Verfahren nach einem der Ansprüche 1-4, wobei die elektrische Schaltung (1) in einem Hörgerät eingebaut ist

6. Verfahren nach Anspruch 5, wobei die dreidimensionale Grafik die Verstärkung (g) oder den Ausgangsschallpegel (Lₒᵤₜ) als Funktion der Frequenz (f) und des Eingangsschallpegels (Lᵢₙ) darstellt.

## Claims

1. Method for adjusting a transmission characteristic of an electronic circuit (1), wherein
a calculation device (2) comprising a display device (3) and a device for selecting, marking and/or modifying (4) is provided,
a data transmission connection (6) is established between the electronic circuit (1) to be adjusted and the calculation device (2),
a current transmission characteristic of the electronic circuit (1) is represented by a graphic on the display device (3),
by means of the device for selecting, marking and/or modifying (4), if and when required, a point (10.1, 10.2, ...) or a segment of the graphic on the display device (3) is selected and the graphically displayed transmission characteristic is modified, as a result of which also an instruction to modify certain operating parameters that define the transmission characteristic is determined, and
the modified operating parameters are transmitted to the electronic circuit (1) and are stored there,
**characterised in that**
the transmission characteristic of the electronic circuit (1) is displayed as a structure (8) in a three-dimensional graphic on the display device (3).

2. Method according to claim 1, wherein the electronic circuit (1) processes an input signal to form an output signal and, in the three-dimensional graphic, a parameter (g, Lout) of the output signal is displayed as a function of two parameters (f, Lᵢₙ) of the input signal.

3. Method according to claim 1 or 2, wherein a target characteristic is predefined and the current transmission characteristic is adapted to this target characteristic in stages.

4. Method according to one of claims 1-3, wherein the three-dimensional graphic is rotated for the purpose of selecting of a suitable view.

5. Method according to one of claims 1-4, wherein the electronic circuit (1) is integrated in a hearing aid.

6. Method according to claim 5; wherein the three-dimensional graphic displays the amplification (g) or the output sound level (Lₒᵤₜ) as a function of the frequency (f) and of the input sound level (Lᵢₙ).

## Revendications

1. Procédé d'ajustement d'une caractéristique de transmission d'un circuit électronique (1), dans lequel
on met. à disposition un dispositif de calcul (2) comportant un dispositif d'affichage (3) et un dispositif de sélection, de marquage et/ou de modification (4),
on réalise une liaison de transmission de données (6) entre le circuit électronique à ajuster (1) et le dispositif de calcul (2),
on représente une caractéristique de transmission réelle du circuit électronique (1) par un graphique sur le dispositif d'affichage (3),
à l'aide du dispositif de sélection, de marquage et/ou de modification (4), on choisit en cas de besoin un point (10.1, 10.2, ...) ou un segment du graphique sur le dispositif d'affichage (3), et on modifie la caractéristique de transmission, représentée par un graphique, ce qui permet aussi de déterminer une instruction pour modifier certains paramètres de marche qui définissent la caractéristique de transmission, et
on transfère les paramètres de marche modifiés au circuit électronique (1) et on les y stocke,
**caractérisé en ce que** la caractéristique de transmission du circuit électronique (1) est représentée sous forme d'une structure (8) dans un graphique tridimensionnel sur le dispositif d'affichage (3).

2. Procédé selon la revendication 1, dans lequel le circuit électronique (1) transforme un signal d'entrée en un signal de sortie, un paramètre (g, Lₒᵤₜ) du signal de sortie étant, dans le graphique tridimensionnel, représenté en fonction de deux paramètres (f, Lᵢₙ) du signal d'entrée.

3. Procédé selon la revendication 1 ou 2, dans lequel une caractéristique cible est prédéfinie, et la caractéristique de transmission réelle de cette caractéristique cible est adaptée pas à pas.

4. Procédé selon l'une des revendications 1 à 3, dans lequel, pour la sélection d'une vue appropriée, on fait tourner le graphique tridimensionnel.

5. Procédé selon l'une des revendications 1 à 4, dans lequel le circuit électronique (1) est incorporé dans un appareil de correction auditive.

6. Procédé selon la revendication 5, dans lequel le graphique tridimensionnel représente le gain (g) ou le niveau acoustique de sortie (Lₒᵤₜ) en fonction de la fréquence (f) et du niveau acoustique d'entrée (Lᵢₙ).
